# EUROPÄISCHE PATENTANMELDUNG

(11) **EP 3 424 489 A1**
(43) Veröffentlichungstag der Anmeldung: **09.01.2019**
(21) Anmeldenummer: 18187486.8
(22) Anmeldetag: 19.12.2012
(51) Int. Cl.: A61K 8/97, A61K 39/395

(54) **ORAL VERABREICHBARE ZUSAMMENSETZUNG BZW. SET UMFASSEND GERBSTOFF**

(30) Priorität: 19.12.2011 AT 500102011
(62) Teilanmeldung aus: 12816646.9
(71) Anmelder: STADA ARZNEIMITTEL AG, 61118 Bad Vilbel (DE)
(72) Erfinder: MISSBICHLER, Albert, 1210 Wien (AT)
(74) Vertreter: Sonn & Partner Patentanwälte

(57) **Zusammenfassung**

Die vorliegende Erfindung betrifft eine Zusammensetzung, umfassend die oral zu verabreichende Kombination von mindestens einem Gerbstoff und mindestens einer Substanz zur Bindung oder Maskierung eines Gluten zugehörigen Proteins, sowie ein Set zur Behandlung einer entzündlichen Erkrankung des Magens und/oder des Darmtrakts umfassend mindestens ein Behältnis mit mindestens einen Gerbstoff und mindestens ein weiteres Behältnis mit mindestens einem Mittel zur Behandlung einer entzündlichen Erkrankung des Magens und/oder des Darmtrakts.

## Beschreibung

Die vorliegende Erfindung betrifft eine Zusammensetzung zur Behandlung von Erkrankungen im Darmtrakt, welche durch Gluten und verwandte Substanzen ausgelöst werden.

Entzündliche Prozesse im Darmbereich können vielfältige Ursachen haben, diese sind in den meisten Fällen schwierig zu diagnostizieren und noch schwieriger zu therapieren. In den letzten Jahren hat die Diagnose der so genannten Lebensmittelunverträglichkeiten einen zunehmend wichtigen Stellenwert in der medizinischen Fachwelt bekommen und damit auch neue Erkenntnisse für die Ursachenerkennung von entzündlichen Prozessen im Darmbereich geliefert.

Viele Menschen entwickeln auch eine Unverträglichkeit gegenüber Histamin und anderen biogenen Aminen. In diesem Fall ist die Leistung des Enzyms Diaminooxidase nicht ausreichend, um die über die Nahrung aufgenommenen biogenen Amine effizient abzubauen. In der Folge gelangt das überschüssige Histamin ins Blut. Typischerweise entwickeln die Betroffenen nach dem Verzehr bestimmter Lebensmittel Kopfschmerzen, Durchfälle, unangenehmen Blähungen und andere allergieähnliche Symptomen.

Sehr komplex stellt sich die Gluten-Unverträglichkeit, auch Zöliakie oder Sprue genannt, dar. Diese Krankheit ist eine vielschichtige Kombination von Resorptionsstörung und interagierender Autoimmunreaktion des Körpers, welche teilweise auch eine genetische HLA-Disposition, nämlich den HLA-DQ2 oder DQ8 Serotyp benötigen. Bislang ist unklar, warum der Großteil der Bevölkerung mit dieser genetischen Disposition einen Toleranzmechanismus entwickelt. Derzeit ist eine Vielzahl von Definitionen von Krankheitsbildern, welche durch Gluten bzw. Gliadin ausgelöst werden, in der Literatur zu finden. Diese umfasst unter anderem:
Asymptomatische Zöliakie (auch Stille Zöliakie): Keine Symptome bei Gluten-Anwesenheit oder Entzug; Antikörper vorhanden; erhöhtes Risiko für Ausbruch einer schweren Zöliakie.
Klassische Zöliakie: Klassische Symptome von Malabsorbtion; Durchfall, Fettstuhl, Gewichtsverlust, Gedeihstörungen. Meist bei Kindern diagnostiziert.
Subklinische Zöliakie: Eher unspezifische Symptome wie Eisenmangel, Leberfunktionsstörungen, ewtl. Biopsieresultate vorhanden.
Symptomatische Zöliakie: Gastrointestinale/extraintestinale Symptome bei Gluten-Einnahme; breites Symptomspektrum.
Refraktäre Zöliakie (RCD): Malabsorbtion & Zottenatrophie nach mehr als 12 Monaten strikter Gluten freier Diät. Serologie meist negativ; positive Serologie weißt auf Diätfehler hin RCD Typ I: Normaler Phänotyp der interepithelialen Lymphocyten RCD Typ II: Ungebremste klonale Expansion von interepithelialen Lymphocyten, Vorstufe zu Enteropathie-assoziiertem Lymphom.
Latente Zöliakie: Positive Serologie ohne histologische Auffälligkeiten des Dünndarms oder normale Histologie der Dünndarmmucosa, die sich auf Glutengabe ändert oder nichtdiagnostizierte Zöliakie oder morphologische Abweichungen der Dünndarmmucosa ohne auffällige Serologie.
Potentielle Zöliakie: Positive Serologie, keine Zottenatrophie.
Celiac Disease Autoimmunity: Positive Serologie, gekennzeichnet durch erhöhte tTG ("tissue transglutaminase"; Gewebetransglutaminase) oder EMA ("Endomysial components of antibodies") Levels an zumindest 2 Messzeitpunken.
Gluten Intoleranz: Synonym für Zöliakie oder eine unspezifische Zöliakie-ähnliche Erkrankung, die auf eine Gluten freie Diät anspricht.
Non-celiac gluten sensitivity (NCGS): Überbegriff immunologischer, morphologischer oder anderer Symptome, die im Zusammenhang mit Gluteneinnahme auftreten (können). Angeborenes Immunsystem ist aktiviert, aber negative Serologie und keine Zottenatrophie. Hier handelt es sich um eine lokale Gluten-Allergie mit nur lokaler IgE Ausschüttung.
Gluten-assoziierten Störungen: Gluten Ataxie, Morbus Duhring (Dermatitis Herpetiformis).

Biochemisch - physiologisch völlig anders gelagert ist die Gluten-Allergie ("Mehl-Allergie"). Dabei handelt es sich um eine klassische IgE-vermittelte Allergie gegen Gluten, wobei die Sensibilisierung der Lunge meist durch Mehlstaub erfolgt.

Die nicht allergische Immunreaktion der Zöliakie wird hauptsächlich durch die alkohollöslichen Proteinfraktionen (so genannte Prolamine) von Weizen (*Gliadin*), Roggen (*Secalin*), Gerste (*Hordein*) und Hafer (*Avenin*) und deren Bruchstücke nach dem peptisch-tryptischen Verdau im Magen ausgelöst.

Die Gesamtheit dieser Proteine wird im Folgenden als Gluten zugehörige Proteine ("gluten related proteins") bezeichnet. Diese Proteine induzieren in vitro und in vivo reproduzierbar das immunologische Reaktionsspektrum der Zöliakie. Sie enthalten charakteristischerweise "Aminosäurewiederholungen", die aus Prolinen und Glutaminen bestehen. Beispielhaft seien die typischen Peptide erwähnt: α-Gliadin: LQLQPF(PQPLPY)₃PQPQPF; γ-Gliadin: FLQPQQPF(PQQ)₂PY(PQQ)₂PFPQ; LMW-Glutenin: QQQQPPFSQQQQSPFSQQQQ; HMW-Glutenin: (GYYPTSPQQ)ₙ. Grundsätzlicher Auslöser des gesamten physiologischen Prozesses der Zöliakie ist die Passage des im Magen anverdauten Glutens durch die epitheliale Zellschicht des Dünndarms in die dahinter liegende Lamina.

Aufgabe der vorliegenden Erfindung ist es Mittel zur Verfügung zu stellen, welche die entzündlichen oder immunologischen Prozesse, welche durch eine oder mehrere der Gluten zugehörigen Proteine ("gluten related proteins") im Darmtrakt ausgelöst werden zu verhindern, zu reduzieren oder zu lindern.

Daher betrifft die vorliegende Erfindung eine Zusammensetzung zur Verwendung zur Behandlung einer Erkrankung des Darmtrakts, welche durch ein Gluten zugehöriges Protein ausgelöst wird, umfassend mindestens ein Mittel, welches an das Gluten zugehörige Protein bindet, dadurch gekennzeichnet, dass die Zusammensetzung gleichzeitig oder maximal innerhalb von 60 (vorzugsweise 50, noch mehr bevorzugt 40, noch mehr bevorzugt 30) Minuten nach Verabreichung mindestens eines Gerbstoffs einem Patienten verabreicht wird.

Erfindungsgemäß wird die Aufgabe durch die Kombination zweier Mittel gelöst, die in zeitlicher Abfolge oral zugeführt werden. Die vorliegende Erfindung stellt erstmals eine funktionelle Kombination von zwei grundlegend unterschiedlichen Wirkungsweisen dar. Zunächst wird die Darmmucosa mit Hilfe von Gerbstoffen stabilisiert und die Resorption potentiell toxischer Substanzen stark reduziert. Gleichzeitig oder unmittelbar anschließend werden die toxischen Substanzen und/oder Mediatoren vermittels spezifischer Adduktoren gebunden und damit dem physiologischen Aufnahmeprozess entzogen.

Eine Aufgabe, nämlich die Bindung oder Maskierung der Gluten zugehörigen Proteine ("gluten related proteins"), wird erfindungsgemäß durch spezifische Antikörper, Antikörperfragmente (z.B. leichte Ketten, Antigen-spezifische Bindungsstellen und dgl.), Aptamere, DARPIN's oder andere geeignete spezifische Bindeproteinstrukturen, die dem Fachmann bekannt sind, erfüllt. Die Herstellung dieser maskierenden Substanzen kann dem jeweiligen Stand der Technik angepasst und optimiert werden.

Bevorzugt sind Antikörper oder deren Fragmente, welche Legehennen im Anschluss an eine Immunisierung mit einem gewünschten Antigen in den Eidotter sezernieren. Diese Immunglobuline (IgY) ähneln in ihrer Struktur den Säuger-Immunglobulinen IgG und IgE, interagieren jedoch auf Grund des Fehlens der FC-Domäne nicht mit dem Säuger-Immunsystem.

Da die Antikörper in natürlicher, bioaktiver Form in den Darm eingebracht werden müssen, bieten sich bevorzugt IgY aus Eidotter an, da hier eine Interaktion mit FC-Rezeptoren von Immunzellen, Rheumatoid Factor, sowie dem Komplementsystem ausgeschlossen werden kann. Dieser Ansatz von spezifischen IgY gegen Gluten wurde bereits durchgeführt (siehe z.B. US 2011/0008362). Alternativ zu den IgY können naturgemäß auch andere, polyklonale oder monoklonale Antikörper oder deren Fragmente eingesetzt werden, wenn sie durch Methoden, welche dem Stand der Technik entsprechen modifiziert und humanverträglich gemacht wurden. Weitere Antikörper im Sinne der Erfindung können aus Echsen oder dem Blut von Lungenfischen gewonnen werden. Weiters bevorzugt im Sinne der Erfindung ist der Einsatz von Aptameren, welche speziell jene Epitope erkennen, welche die unerwünschten Reaktionen im Magen-Darm-Bereich auslösen oder steuern. Die Entwicklung und Charakterisierung dieser Aptamere ist dem Fachmann bekannt.

Der Begriff "Gluten zugehörige Proteine" ("gluten related proteins"), wie hierin verwendet, umfasst Gliadin, Secalin, Hordein und Avenin und deren Bruchstücke nach dem peptisch-tryptischen Verdau im Magen, wobei besonders bevorzugt Gliadin und dessen Bruchstücke sind. Daher kann als Synonym für "Gluten zugehörige Proteine" "Gliadin, Secalin, Hordein und Avenin und deren Bruchstücke nach dem peptisch-tryptischen Verdau im Magen", "Gliadin, Secalin, Hordein und Avenin und durch Pepsin und/oder Trypsin daraus hergestellte Bruchstücke (Fragmente)" oder "Gliadin, Secalin, Hordein, Avenin und Fragmente davon" verwendet werden. Fragmente im Sinne der vorliegenden Erfindung bestehen aus 5 bis 100, vorzugsweise 10 bis 50, Aminosäuren.

Die Gerbstoffe erfüllen die Aufgabe der Stabilisierung der Darmmucosa. Gerbstoffe stellen eine weite Familie von verwandten, polyphenolischen Substanzen dar, welche in höheren Pflanzen und Algen vorkommen. Sie sind im Bausteinprinzip verzweigte Makromoleküle, die gewisse physikalisch-chemische Eigenschaften teilen: Wasserlöslichkeit, und die Fähigkeit, sich an Proteine anzulagern und diese zu vernetzen, Bildung von Niederschlägen mit Alkaloiden sowie die Bindung von Chelatkomplexen mit Fe^{III}-Salzen. Erfindungsgemäß können unterschiedliche Gerbstoffe, auch in gemischter Form, für die Stabilisierung der Mucosa eingesetzt werden. Bevorzugt werden Gerbstoffe der Typen Catechingerbstoffe, Tannine, Lamiaceen-Gerbstoffe oder Algen-Gerbstoffe eingesetzt. Quellen für Gerbstoffen sind z.B: Galläpfel (Gallae); Hamamelisblätter (Hamamelidis folium); Walnussblätter (Juglandis folium; Eichenrinde (Quercus cortex); Ratanhiawurzel (Ratanhiae radix); Blutwurzwurzel (Tormentillae rhizoma); Heidelbeeren (Myrtilli fructus); Catechu; Rubus fruticosus; Potentilla anserina; Fragaria vesca; Agrimonia eupatoria; Alchemilla xanthochlora; Plantago major; Plantago lanceolata, Rosa gallica; Sanguisorba officinalis oder auch Kerne von Datteln, Trauben usw. Fakultativ können erfindungsgemäß auch synthetische Gerbstoffe wie z.B. Polymere von Acrylaten, Polyurethanen, Isocyanaten, Derivate von Aldehyden aber auch Salze von Mineralstoffen wie z.B: Alaune, Chromsalze oder Zirkonsalze eingesetzt werden.

Gemäß einer besonders bevorzugten Ausführungsform ist der mindestens eine Gerbstoff ausgewählt aus der Gruppe bestehend aus Catechingerbstoffe, Tannine, Lamiaceen-Gerbstoffe und Algen-Gerbstoffe.

Die Fähigkeit, Proteine zu vernetzen beruht auf einer unspezifischen Anlagerung der phenolischen Strukturen an hydrophobe Taschen, sowie der Ausbildung von Wasserstoffbrücken zwischen den Hydroxylgruppen des Gerbstoffes und den Seitenketten polarer Aminosäuren. Die Anlagerung der Gerbstoffe führt zu einer Auffaltung und bei einer ausreichenden Konzentration zur Quervernetzung der Proteine.

Besonders bevorzugt werden die Gerbstoffe eingesetzt, nachdem sie mit Protein im Verhältnis 20:80 [Gerbstoff:Protein]; bevorzugt 35:65, besonders bevorzugt 50:50 vorgefällt (z.B. durch Inkontaktbringen des Gerbstoffs mit dem Protein/Polypeptid) werden. Als Protein können generell Proteine mit einer Molekularmasse zwischen 50 kDa und 500 kDa eingesetzt werden

Überaschenderweise kann durch die Vorfällung des Gerbstoffes mit einem Antikörper oder Bindeprotein ein doppelter Effekt erzielt werden: das entstehende Makromolekül vereinigt die Fähigkeit der Vernetzung der Darmmucosa mit der gleichzeitigen Fähigkeit, toxische Antigene und Substanzen zu binden und deren Bioverfügbarkeit zu reduzieren. Ganz besonders bevorzugt ist daher der Einsatz von Bindeproteinstrukturen, welche mit einem Gerbstoff im Verhältnis 20:80 [Gerbstoff:Protein]; bevorzugt 35:65, besonders bevorzugt 50:50 vorgefällt werden.

Daher betrifft die vorliegende Erfindung eine Zusammensetzung umfassend die oral zu verabreichende Kombination von mindestens einem Gerbstoff und mindestens einer Substanz zur Bindung oder Maskierung der Gluten zugehörigen Proteine ("gluten related proteins") mit dem Ziel der Behandlung von entzündlichen oder nicht allergischen immunologischen Prozessen, welche durch eine oder mehrere der Gluten zugehörigen Proteine ("gluten related proteins") im Darmtrakt ausgelöst werden.

Zusätzlich können neben der Substanz zur Bindung oder Maskierung der Gluten zugehörigen Proteine ("gluten related proteins") und dem Gerbstoff auch bioaktive Mikroorganismen verabreicht werden, die den Abbau dieser toxischen Substanzen und/oder Mediatoren unterstützen und somit potentielle weitere pathologische Prozesse unterbinden.

In einer weiteren Ausformung der erfindungsgemäßen Zusammensetzung können die spezifisch gebundenen toxischen Substanzen, Peptide bzw. Antigene oder Mediatoren, welche mit dem Speisebrei im Darm weiterbewegt wurden, in weiterer Folge durch die Gabe von speziellen bakteriellen Proteasen völlig abgebaut werden. Im Falle von Zöliakie ist das Enzym der Wahl die in allen Lebewesen vorhandene Prolylendopeptidase (PEP; EC 3.4.21.26). Sie kann Peptide, welche aus Gluten und Gliadin entstehen, als einziges Enzym einem bestimmten Ausmass abauen. PEP kommt primär als intrazelluläres Enzym vor, einige Microorganismen, z.B. Flavobacterium meningosepticum, A. niger, sowie diverse Lactobacilli, sind imstande, dieses Enzym auch zu sezernieren. Weitere erfindungsgemäße Mikroorganismen sind unter anderem oder Bakterien aus dem menschlichen Mundraum wie z.B. Fusobakterien oder Actinomyceten.

Die Verabreichung der Bindeproteinstrukturen und Gerbstoffe erfolgt vorzugsweise in einem definierten zeitlichen Abstand: zunächst wird in einem Zeitraum von 0 bis 60 min, bevorzugt 10 bis 40 min, besonders bevorzugt 15 bis 30 min vor Einnahme der glutenhaltigen Nahrung das Gerbstoffpräparat oral verabreicht Gemäß einer bevorzugten Ausführungsform der vorliegenden Erfindung ist der Antikörper, Antikörperfragment, Aptamer und/oder DARpin gegen Gliadin und/oder Fragmenten davon, insbesondere gegen tryptisch und/oder peptisch gespaltenes Gliadin, oder physiologisch äquivalente Verdaue von Gluten oder Gluten-Fraktionen gerichtet.

Das mindestens eine Protein oder Polypeptid, insbesondere der mindestens eine Antikörper, ist vorzugsweise rekombinanten Ursprungs.

Gemäß einer noch weiteren bevorzugten Ausführungsform der vorliegenden Erfindung ist der mindestens eine Antikörper ein polyklonaler Antikörper aus Säugetieren, polyklonaler Antikörper avianen Ursprungs, ein Antikörper aus Echsen, ein monoklonaler Antikörper oder ein Antikörper aus dem Blut von Lungenfischen.

Gemäß einer bevorzugten Ausführungsform der vorliegenden Erfindung umfasst die erfindungsgemäße Zusammensetzung eukaryotische und/oder prokaryotische Zellen, die in der Lage sind entzündliche Prozesse im Magen und/oder Darmtrakts zu reduzieren und/oder zu unterbinden.

Die in der erfindungsgemäßen Zusammensetzung vorhandenen Zellen sind vorzugsweise in der Lage Proteine (wie zum Beispiel Enzyme) zu sekretieren, die in der Lage sind entzündliche Prozesse im Darmtrakt zu reduzieren bzw. zu unterbinden, die für die Bildung einer entzündlichen Erkrankung verantwortlich sind.

Gemäß einer besonders bevorzugten Ausführungsform wird die Zusammensetzung umfassend mindestens einen Gerbstoff zur Behandlung einer entzündlichen Erkrankung des Darmtrakts, in Kombination mit mindestens einem zweiten Mittel zur Behandlung einer entzündlichen Erkrankung des Darmtrakts verabreicht, wobei das mindestens eine zweite Mittel mindestens ein Protein oder Polypeptid, welches mindestens eine Substanz (aktiv) bindet, welches die entzündliche Erkrankung induziert oder mediiert, und eukaryotische und/oder prokaryotische Zellen umfasst, die in der Lage sind entzündliche Prozesse im Magen und/oder Darmtrakts zu reduzieren und/oder zu unterbinden. D.h. die erfindungsgemäße Zusammensetzung umfassend mindestens einen Gerbstoff wird in Kombination mit einer Zusammensetzung umfassend zumindest ein Protein oder Polypeptid und mit einer Zusammensetzung umfassend eukaryotische und/oder prokaryotische Zellen, beides wie oben definiert, verabreicht. Die beiden zuletzt genannten Zusammensetzungen können entweder in einer oder mindestens zwei einzelnen Darreichungsformen zur Verabreichung bereitgestellt werden.

Gemäß einer bevorzugten Ausführungsform der vorliegenden Erfindung sind die eukaryotischen und/oder prokaryotischen Zellen ausgewählt aus der Gruppe bestehend aus Flavobacterium sp, Lactobacillus sp, Aspergillus sp und Bifidobacterium sp.

Der mindestens eine Gerbstoff ist vorzugsweise mit mindestens einem Protein vorgefällt.

Gemäß einer weiteren bevorzugten Ausführungsform der vorliegenden Erfindung liegt das mindestens eine Mittel, welches an das Gluten zugehörige Protein bindet, und der mindestens eine Gerbstoff in einer Formulierung zur kontrollierten Freisetzung im Magen und/oder Darmtrakt, vorzugsweise Darmtrakt, vor.

Dem Fachmann sind Mittel zum Formulieren von Wirkstoffen bekannt, die es ermöglichen eine Zusammensetzung bereitzustellen, welche die Eigenschaft aufweist unter bestimmten Bedingungen die in der Zusammensetzung eingearbeiteten Wirkstoffe freizusetzen. Pharmazeutisch werden derartige Mittel dazu eingesetzt und Wirkstoffe zum Beispiel ausschließlich Darmtrakt freizusetzen. Dies hat den Vorteil, dass die Wirkstoffe magensaftresistent durch den Magen in den Darm geschleust werden können.

Der Begriff "Formulierung zur kontrollierten Freisetzung" umfasst nicht nur magensaftresistente Darreichungsformen bzw. Formulierungen sondern auch "Delayed release" zur Freisetzung im Darm mit Zeitverzögerung, Zuckerabbaubare Polymerüberzüge zur Freisetzung im Dickdarm, Überzug als Gelbarriere zur zielgerichteten Freisetzung im Darm je nach Dicke der Barriere, Wasserunlösliche Überzüge mit Porenbildner retardiert die Freisetzung je nach Porengrüße und Menge, pH-sensitive Überzüge zur Freisetzung entsprechend dem physiologischen pH-Gradienten, bioadhärente Überzüge (Chitosan, Polyacrylate) interagieren mit dem Mucos und halten die Arzneiform fest, flotierende Arzneiformen zur Freisetzung im Magen dadurch, dass im Magen Quellung eintritt und der Durchtritt durch den Pylorus verhindert wird, Verarbeitung in Pellets wenn kleiner als 1 bis 3 mm für einen raschen Durchtritt der Wirkstoffe durch den Pylorus und Transfer in den Darm.

Es hat sich erfindungsgemäß als besonders vorteilhaft erwiesen, dass die Gerbstoffe vor der erfindungsgemäßen Zusammensetzung umfassend das mindestens eine Mittel eine Mittel, welches an das Gluten zugehörige Protein bindet, verabreicht wird, da dadurch die Benetzung der Schleimhäute, insbesondere Darmschleimhäute, mit den erfindungsgemäß eingesetzten Gerbstoffen vor dem Eintreffen der zuvor genannten Mittel erfolgt. Dadurch wird es ermöglicht, zunächst die Schleimhäute zu stabilisieren, woraufhin die eigentlichen Wirkstoffe zur Behandlung der entzündlichen Erkrankung verabreicht werden.

Die Zusammensetzung umfassend mindestens einen Gerbstoff wird daher mindestens eine, vorzugsweise mindestens fünf, noch mehr bevorzugt mindestens zehn, am meisten bevorzugt mindestens 20, Minuten vor dem mindestens einem Mittel zur Behandlung einer entzündlichen Erkrankung des Darmtrakts verabreicht.

Gemäß einer bevorzugten Ausführungsform der vorliegenden Erfindung wird der mindestens eine Gerbstoff in einer Menge von 10 bis 10000 mg, vorzugsweise von 20 bis 5000 mg, noch mehr bevorzugt von 50 bis 2500 mg, besonders bevorzugt von 100 bis 2000 mg, verabreicht.

Gemäß einer weiteren bevorzugten Ausführungsform der vorliegenden Erfindung wird das mindestens eine Protein oder Polypeptid, welches an mindestens eine Substanz (z.B. Antigen) bindet, welche die Gluten-Unverträglichkeit induziert oder mediiert, in einer Menge von 1 bis 100000 mg, vorzugsweise von 5 bis 50.000 mg, noch mehr bevorzugt von 10 bis 20.000 mg, besonders bevorzugt von 15 bis 15.000 mg, verabreicht.

Gemäß einer besonders bevorzugten Ausführungsform der vorliegenden Erfindung, werden die eukaryotischen und/oder prokaryotischen Zellen in einer Menge von 10⁹ bis 10¹⁴, vorzugsweise von 10 bis 10¹³, verabreicht.

Die Verabreichung der erfindungsgemäß eingesetzten Bindeproteinstrukturen und Gerbstoffe erfolgt erfindungsgemäß in einem definierten zeitlichen Abstand: zunächst wird in einem Zeitraum von 0 - 60 min, bevorzugt 10 bis 40 min, besonders bevorzugt 15 bis 30 min vor Einnahme der glutenhaltigen Nahrung das Gerbstoffpräparat oral verabreicht

Bei oraler Verabreichung der erfindungsgemäßen Zusammensetzungen werden diese entsprechend formuliert (zum Beispiel magensaftresistent) .

Ein noch weiterer Aspekt der vorliegenden Erfindung betrifft ein Set zur Verwendung zur Behandlung einer Erkrankung des Darmtrakts, welche durch ein Gluten zugehöriges Protein ausgelöst wird, umfassend mindestens ein Behältnis mit mindestens einen Gerbstoff und mindestens ein weiteres Behältnis mit der erfindungsgemäßen Zusammensetzung.

Gemäß einer bevorzugten Ausführungsform der vorliegenden Erfindung umfasst das Set Zusammensetzungen wie oben definiert und wird zu den oben angeführten Zwecken eingesetzt und der oben beschriebenen Art verabreicht.

Weitere Ausführungsformen:
1. Zusammensetzung umfassend mindestens einen Gerbstoff zur Behandlung einer entzündlichen Erkrankung des Magens und/oder des Darmtrakts, wobei die Zusammensetzung in Kombination mit mindestens einem zweiten Mittel zur Behandlung einer entzündlichen Erkrankung des Magens und/oder des Darmtrakts verabreicht wird.
2. Zusammensetzung gemäß Ausführungsform 1, dadurch gekennzeichnet, dass die entzündliche Erkrankung des Magens und/oder des Darmtrakts ausgewählt ist aus der Gruppe bestehend aus Zöliakie, Morbus Crohn, Colitis, insbesondere Colitis cystica, Colitis haemorrhagica, Colitis ischaemia, Colitis pseudomembranacea oder Colitis ulcerosa, Reizdarm und Gastritis.
3. Zusammensetzung gemäß Ausführungsform 1 oder 2, dadurch gekennzeichnet, dass das mindestens eine zweite Mittel zur Behandlung einer entzündlichen Erkrankung des Magens und/oder des Darmtrakts mindestens ein Protein oder Polypeptid umfasst, welches mindestens eine Substanzbindet, welche die entzündliche Erkrankung induziert oder mediiert.
4. Zusammensetzungen gemäß Ausführungsform 3, dadurch gekennzeichnet, dass das mindestens eine Protein oder Polypeptid ein Antikörper, ein Aptamer und/oder ein DARpin ("*Designed Ankyrin Repeat Proteins*") gerichtet gegen eine Substanz, welche die entzündliche Erkrankung induziert oder mediiert, oder ein spezifischer Rezeptor gerichtet gegen einen Wirkstoff, welcher die entzündliche Erkrankung induziert oder mediiert, ist.
5. Zusammensetzung gemäß Ausführungsform 4, dadurch gekennzeichnet, dass der mindestens eine Antikörper, Aptamer und/oder DARpin gegen Gliadin und/oder Fragmenten davon, insbesondere gegen tryptisch und/oder peptisch gespaltenes Gliadin, oder physiologisch äquivalente Verdaue von Gluten oder Gluten-Fraktionen gerichtet ist.
6. Zusammensetzung gemäß einer der Ausführungsformen 3 oder 5, dadurch gekennzeichnet, dass das mindestens ein Protein oder Polypeptid, insbesondere der mindestens eine Antikörper, rekombinanten Ursprungs ist.
7. Zusammensetzung gemäß Ausführungsform 4 oder 5, dadurch gekennzeichnet, dass der mindestens eine Antikörper ein polyklonaler Antikörper aus Säugetieren, polyklonaler Antikörper avianen Ursprungs, ein Antikörper aus Echsen, ein monoklonaler Antikörper oder ein Antikörper aus dem Blut von Lungenfischen.
8. Zusammensetzung gemäß einer der Ausführungsformen 1 bis 5, dadurch gekennzeichnet, dass das mindestens eine Mittel zur Behandlung einer entzündlichen Erkrankung des Magens und/oder des Darmtrakts eukaryotische und/oder prokaryotische Zellen umfasst, die in der Lage sind entzündliche Prozesse im Magen und/oder Darmtrakts zu reduzieren und/oder zu unterbinden.
9. Zusammensetzung gemäß Ausführungsform 8, dadurch gekennzeichnet, dass die eukaryotischen und/oder prokaryotischen Zellen ausgewählt sind aus der Gruppe bestehend aus Flavobacterium sp, Lactobacillus sp, Aspergillus sp und Bifidobacterium sp
10. Zusammensetzung gemäß einer der Ausführungsformen 1 bis 9, dadurch gekennzeichnet, dass der mindestens eine Gerbstoff ausgewählt ist aus der Gruppe bestehend aus Catechingerbstoffe, Tannine, Lamiaceen-Gerbstoffe und Algen-Gerbstoffe.
11. Zusammensetzung gemäß einer der Ausführungsformen 1 bis 8, dadurch gekennzeichnet, dass der mindestens eine Gerbstoff mit mindestens einem Protein vorgefällt ist.
12. Zusammensetzung gemäß Ausführungsform 11, dadurch gekennzeichnet, dass das mindestens eine Protein ein Antikörper ist, wie in einer der Ausführungsformen 4 bis 7 definiert.
13. Zusammensetzung gemäß einer der Ausführungsformen 1 bis 10, dadurch gekennzeichnet, dass der mindestens eine Gerbstoff und das mindestens eine Mittel zur Behandlung einer entzündlichen Erkrankung des Magens und/oder des Darmtrakts in einer Formulierung zur kontrollierten Freisetzung im Magen und/oder Darmtrakt, vorzugsweise Darmtrakt, vorliegt.
14. Zusammensetzung gemäß einer der Ausführungsformen 1 bis 13, dadurch gekennzeichnet, dass die Zusammensetzung umfassend mindestens einen Gerbstoff gleichzeitig und/oder vor dem mindestens einem Mittel zur Behandlung einer entzündlichen Erkrankung des Magens und/oder des Darmtrakts verabreicht wird.
15. Zusammensetzung gemäß einer der Ausführungsformen 1 bis 14, dadurch gekennzeichnet, dass die Zusammensetzung umfassend mindestens einen Gerbstoff mindestens eine, vorzugsweise mindestens fünf, noch mehr bevorzugt mindestens zehn, am meisten bevorzugt mindestens 20, Minuten vor dem mindestens einem Mittel zur Behandlung einer entzündlichen Erkrankung des Magens und/oder des Darmtrakts verabreicht wird.
16. Zusammensetzung gemäß einer der Ausführungsformen 1 bis 15, dadurch gekennzeichnet, dass der mindestens eine Gerbstoff in einer Menge von 100 bis 10000 mg, vorzugsweise von 100 bis 5000 mg, noch mehr bevorzugt von 100 bis 2500 mg, besonders bevorzugt von 500 bis 2000 mg, verabreicht wird.
17. Zusammensetzung gemäß einer der Ausführungsformen 3 bis 7 und 10 bis 16, dadurch gekennzeichnet, dass das mindestens eine Protein oder Polypeptid, welches an mindestens eine Substanz bindet, welches die entzündliche Erkrankung induziert oder mediiert, in einer Menge von 200 bis 100000 mg, vorzugsweise von 200 bis 50.000 mg, noch mehr bevorzugt von 200 bis 20.000 mg, besonders bevorzugt von 500 bis 15.000 mg, verabreicht wird.
18. Zusammensetzung gemäß einer der Ausführungsformen 8 bis 17, dadurch gekennzeichnet, dass die eukaryotischen und/oder prokaryotischen Zellen in einer Menge von 10⁹ bis 10¹⁴, vorzugsweise von 10¹⁰ bis 10¹³, verabreicht werden.
19. Set zur Behandlung einer entzündlichen Erkrankung des Magens und/oder des Darmtrakts umfassend mindestens ein Behältnis mit mindestens einen Gerbstoff und mindestens ein weiteres Behältnis mit mindestens einem Mittel zur Behandlung einer entzündlichen Erkrankung des Magens und/oder des Darmtrakts.
20. Set gemäß Ausführungsform 19 wie in einer der Ausführungsformen 1 bis 18 definiert.

B1. Zusammensetzung zur Verwendung zur Behandlung einer Erkrankung des Darmtrakts, welche durch ein Gluten zugehöriges Protein ausgelöst wird, umfassend mindestens ein Mittel, welches an das Gluten zugehörige Protein bindet, dadurch gekennzeichnet, dass die Zusammensetzung gleichzeitig oder maximal innerhalb von 60 Minuten nach Verabreichung mindestens eines Gerbstoffs einem Patienten verabreicht wird.
B2. Zusammensetzung gemäß Ausführungsform B1, dadurch gekennzeichnet, dass die Erkrankung des Darmtrakts, welche durch eine oder mehrere Gluten zugehörigen Proteine ausgelöst wird Zöliakie ist.
B3. Zusammensetzung gemäß Ausführungsform B2, dadurch gekennzeichnet, dass die Zöliakie ausgewählt ist aus der Gruppe bestehend aus asymptomatischer Zöliakie, klassischer Zöliakie, subklinischer Zöliakie, symptomatischer Zöliakie, refraktärer Zöliakie, latenter zöliakie, potentieller Zöliakie, Celiac Disease Autoimmunity, Gluten-Intoleranz, Non-celiac gluten sensitivity und Gluten-assoziierten Störungen.
B4. Zusammensetzung gemäß einer der Ausführungsformen B1 bis B3, dadurch gekennzeichnet, dass das mindestens eine Mittel, welches an das Gluten zugehörige Protein bindet, ein Protein oder Polypeptid umfasst.
B5. Zusammensetzungen gemäß einer der Ausführungsformen B1 bis B4, dadurch gekennzeichnet, dass das mindestens eine Mittel, welches an das Gluten zugehörige Protein bindet, ein Antikörper, ein Antikörperfragment, ein Aptamer und/oder ein DARpin ("*Designed Ankyrin Repeat Proteins*") oder ein spezifischer Rezeptor gerichtet gegen das Gluten zugehörige Protein ist.
B6. Zusammensetzung gemäß einer der Ausführungsformen B1 bis B5, dadurch gekennzeichnet, dass das Gluten zugehöriges Protein ausgewählt ist aus der Gruppe bestehend aus Gliadin, Secalin, Hordein, Avenin und Fragmente davon.
B7. Zusammensetzung gemäß einer der Ausführungsformen B1 bis B6, dadurch gekennzeichnet, dass das Gluten zugehörige Protein Gliadin oder ein Fragment davon ist.
B8. Zusammensetzung gemäß einer der Ausführungsformen B5 bis B7, dadurch gekennzeichnet, dass der mindestens eine Antikörper ein polyklonaler Antikörper aus Säugetieren, polyklonaler Antikörper avianen Ursprungs, ein Antikörper aus Echsen, ein monoklonaler Antikörper oder ein Antikörper aus dem Blut von Lungenfischen.
B9. Zusammensetzung gemäß einer der Ausführungsformen B1 bis B5, dadurch gekennzeichnet, dass die Zusammensetzung eukaryotische und/oder prokaryotische Zellen umfasst, die in der Lage sind entzündliche Prozesse im Magen und/oder Darmtrakts zu reduzieren und/oder zu unterbinden.
B10. Zusammensetzung gemäß Ausführungsform B9, dadurch gekennzeichnet, dass die eukaryotischen und/oder prokaryotischen Zellen ausgewählt sind aus der Gruppe bestehend aus Flavobacterium sp, Lactobacillus sp, Aspergillus sp und Bifidobacterium sp
B11. Zusammensetzung gemäß einer der Ausführungsformen B1 bis B9, dadurch gekennzeichnet, dass der mindestens eine Gerbstoff ausgewählt ist aus der Gruppe bestehend aus Catechingerbstoffe, Tannine, Lamiaceen-Gerbstoffe und Algen-Gerbstoffe.
B12. Zusammensetzung gemäß einer der Ausführungsformen B1 bis B8, dadurch gekennzeichnet, dass der mindestens eine Gerbstoff mit mindestens einem Protein vorgefällt ist.
B13. Zusammensetzung gemäß Ausführungsform B12, dadurch gekennzeichnet, dass das mindestens eine Protein ein Antikörper ist, wie in einem der Ausführungsformen B5 bis B8 definiert.
B14. Zusammensetzung gemäß einer der Ausführungsformen B1 bis B13, dadurch gekennzeichnet, dass das mindestens eine Mittel, welches an das Gluten zugehörige Protein bindet, und der mindestens eine Gerbstoff in einer Formulierung zur kontrollierten Freisetzung im Magen und/oder Darmtrakt, vorzugsweise Darmtrakt, vorliegt.
B15. Zusammensetzung gemäß einer der Ausführungsformen B1 bis B14, dadurch gekennzeichnet, dass der mindestens eine Gerbstoff mindestens eine, vorzugsweise mindestens fünf, noch mehr bevorzugt mindestens zehn, am meisten bevorzugt mindestens 20, Minuten vor der Zusammensetzung umfassend das mindestens eine Mittel, welches an das Gluten zugehörige Protein bindet, verabreicht wird.
B16. Zusammensetzung gemäß einer der Ausführungsformen B1 bis B15, dadurch gekennzeichnet, dass der mindestens eine Gerbstoff in einer Menge von 100 bis 10000 mg, vorzugsweise von 100 bis 5000 mg, noch mehr bevorzugt von 100 bis 2500 mg, besonders bevorzugt von 500 bis 2000 mg, verabreicht wird.
B17. Zusammensetzung gemäß einer der Ausführungsformen B1 bis B16, dadurch gekennzeichnet, dass das Mittel, welches an das Gluten zugehörige Protein bindet, in einer Menge von 200 bis 100000 mg, vorzugsweise von 200 bis 50.000 mg, noch mehr bevorzugt von 200 bis 20.000 mg, besonders bevorzugt von 500 bis 15.000 mg, verabreicht wird.
B18. Zusammensetzung gemäß einer der Ausführungsformen B9 bis B17, dadurch gekennzeichnet, dass die eukaryotischen und/oder prokaryotischen Zellen in einer Menge von 10⁹ bis 10¹⁴, vorzugsweise von 10¹⁰ bis 10¹³, verabreicht werden.
B19. Set zur Verwendung zur Behandlung einer Erkrankung des Darmtrakts, welche durch ein Gluten zugehöriges Protein ausgelöst wird, umfassend mindestens ein Behältnis mit mindestens einen Gerbstoff und mindestens ein weiteres Behältnis mit der Zusammensetzung wie in einem der Ausführungsformen B1 bis B18 definiert.

## Patentansprüche

1. Zusammensetzung, umfassend die oral zu verabreichende Kombination von mindestens einem Gerbstoff und mindestens einer Substanz zur Bindung oder Maskierung eines Gluten zugehörigen Proteins.

2. Zusammensetzung gemäß Anspruch 1, **dadurch gekennzeichnet, dass** die mindestens eine Substanz ein Protein oder Polypeptid umfasst.

3. Zusammensetzung gemäß Anspruch 1 oder 2, **dadurch gekennzeichnet, dass** die mindestens eine Substanz ein Antikörper, ein Antikörperfragment, ein Aptamer und/oder ein DARpin ("Designed Ankyrin Repeat Proteins") oder ein spezifischer Rezeptor gerichtet gegen das Gluten zugehörige Protein ist.

4. Zusammensetzung gemäß einem der Ansprüche 1 bis 3, **dadurch gekennzeichnet, dass** das Gluten zugehörige Protein ausgewählt ist aus der Gruppe bestehend aus Gliadin, Secalin, Hordein, Avenin und Fragmenten davon.

5. Zusammensetzung gemäß Anspruch 4, **dadurch gekennzeichnet, dass** das Gluten zugehörige Protein Gliadin oder ein Fragment davon ist.

6. Zusammensetzung gemäß einem der Ansprüche 3 bis 5, **dadurch gekennzeichnet, dass** der mindestens eine Antikörper ein polyklonaler Antikörper aus Säugetieren, polyklonaler Antikörper avianen Ursprungs, ein Antikörper aus Echsen, ein monoklonaler Antikörper oder ein Antikörper aus dem Blut von Lungenfischen ist.

7. Zusammensetzung gemäß einem der Ansprüche 1 bis 6, **dadurch gekennzeichnet, dass** die Zusammensetzung eukaryotische und/oder prokaryotische Zellen umfasst, die in der Lage sind, entzündliche Prozesse im Magen und/oder Darmtrakts zu reduzieren und/oder zu unterbinden; vorzugsweise wobei die eukaryotischen und/oder prokaryotischen Zellen ausgewählt sind aus der Gruppe bestehend aus Flavobacterium sp, Lactobacillus sp, Aspergillus sp und Bifidobacterium sp.

8. Zusammensetzung gemäß einem der Ansprüche 1 bis 7, **dadurch gekennzeichnet, dass** der mindestens eine Gerbstoff ausgewählt ist aus der Gruppe bestehend aus Catechingerbstoffen, Tanninen, Lamiaceen-Gerbstoffen und Algen-Gerbstoffen; und/oder wobei der mindestens eine Gerbstoff mit mindestens einem Protein vorgefällt ist.

9. Zusammensetzung gemäß einem der Ansprüche 1 bis 8, **dadurch gekennzeichnet, dass** die mindestens eine Substanz, und der mindestens eine Gerbstoff in einer Formulierung zur kontrollierten Freisetzung im Magen und/oder Darmtrakt, vorzugsweise Darmtrakt, vorliegen.

10. Set zur Behandlung einer entzündlichen Erkrankung des Magens und/oder des Darmtrakts umfassend mindestens ein Behältnis mit mindestens einen Gerbstoff und mindestens ein weiteres Behältnis mit mindestens einem Mittel zur Behandlung einer entzündlichen Erkrankung des Magens und/oder des Darmtrakts.

11. Set gemäß Anspruch 10, **dadurch gekennzeichnet, dass** das mindestens eine Mittel mindestens ein Protein oder Polypeptid umfasst, welches mindestens eine Substanz bindet, welche die entzündliche Erkrankung induziert oder mediiert.

12. Set gemäß Anspruch 11, **dadurch gekennzeichnet, dass** das mindestens eine Protein oder Polypeptid ein Antikörper, ein Antikörperfragment, ein Aptamer und/oder ein DARpin ("Designed Ankyrin Repeat Proteins") gerichtet gegen eine Substanz, welche die entzündliche Erkrankung induziert oder mediiert, oder ein spezifischer Rezeptor gerichtet gegen einen Wirkstoff, welcher die entzündliche Erkrankung induziert oder mediiert, ist.

13. Set gemäß Anspruch 12, **dadurch gekennzeichnet, dass** der mindestens eine Antikörper, das mindestens eine Antikörperfragment, Aptamer und/oder DARpin gegen Gliadin und/oder Fragmente davon, insbesondere gegen tryptisch und/oder peptisch gespaltenes Gliadin, oder physiologisch äquivalente Verdaue von Gluten oder Gluten-Fraktionen, gerichtet ist.

14. Set gemäß einem der Ansprüche 10 bis 13, **dadurch gekennzeichnet, dass** der mindestens eine Gerbstoff ausgewählt ist aus der Gruppe bestehend aus Catechingerbstoffe, Tannine, Lamiaceen-Gerbstoffe und Algen-Gerbstoffe.

15. Set gemäß einem der Ansprüche 10 bis 14, **dadurch gekennzeichnet, dass** der mindestens eine Gerbstoff mit mindestens einem Protein vorgefällt ist.
